(19) Europäisches Patentamt
European Patent Office
Office européen des brevets

(11) **EP 4 502 154 A2**

(12) **EUROPEAN PATENT APPLICATION**
published in accordance with Art. 153(4) EPC

(43) Date of publication:
**05.02.2025 Bulletin 2025/06**

(21) Application number: **24735118.2**

(22) Date of filing: **01.03.2024**

(51) International Patent Classification (IPC):
**C12N 9/12** $^{(2006.01)}$       **C12N 15/70** $^{(2006.01)}$
**C12N 15/52** $^{(2006.01)}$

(52) Cooperative Patent Classification (CPC):
**C12N 9/12**

(86) International application number:
**PCT/CN2024/079536**

(87) International publication number:
**WO 2024/131998 (27.06.2024 Gazette 2024/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: 01.03.2023  CN 202310184997
09.05.2023  CN 202310515121
15.08.2023  CN 202311023160
20.09.2023  CN 202311214811
01.02.2024  CN 202410141858

(71) Applicant: **Nanjing Vazyme Biotech Co., Ltd.**
**Nanjing, Jiangsu 210046 (CN)**

(72) Inventors:
• **XU, Xiaoyu**
**Nanjing, Jiangsu 210046 (CN)**
• **JIN, Qiuheng**
**Nanjing, Jiangsu 210046 (CN)**
• **HE, Wei**
**Nanjing, Jiangsu 210046 (CN)**
• **WANG, Chong**
**Nanjing, Jiangsu 210046 (CN)**
• **HU, Huixue**
**Nanjing, Jiangsu 210046 (CN)**

(74) Representative: **Ostertag & Partner Patentanwälte mbB**
**Azenbergstraße 35**
**70174 Stuttgart (DE)**

Remarks:
The complete document including Reference Table(s) and the Sequence Listing(s) can be downloaded from the EPO website

(54) **RNA POLYMERASE VARIANT, AND PREPARATION METHOD THEREFOR AND USE THEREOF IN RNA SYNTHESIS**

(57)    Provided are an RNA polymerase variant and a preparation method therefor. When the RNA polymerase variant is used in in-vitro transcription, an RNA product with low dsRNA contamination and a high integrity can be obtained, the utilization efficiency of cap analogs in the co-transcriptional capping reaction system can also be improved, and costs are saved. In addition, further provided is a method for generating RNA by means of in-vitro transcription.

EP 4 502 154 A2

## Description

### TECHNICAL FIELD

The present application belongs to the field of biotechnology, and particularly relates to an RNA polymerase variant, a preparation method therefor, and the use thereof in RNA synthesis.

### BACKGROUND ART

The global outbreak of Corona Virus Disease 2019 (COVID-19) in late 2019 has caused billions of infections in people worldwide. Before Paxlovid (nirmatrelvir tablets/ritonavir tablets (co-packaged)) was available on the market (Emergency Use Authorization by FDA on November 22, 2021), vaccines became the most effective line of defense against infection. In this massive global fight against the epidemic, billions of people have been vaccinated with COVID-19 vaccines such as BNT162b2 (mRNA vaccine) developed by BioNTech and Pfizer, mRNA-1273 (mRNA vaccine) developed by Moderna, or AZD1222 (adenovirus vector vaccine) developed by AstraZeneca, among which RNA vaccines have the highest protective efficacy. mRNA vaccines have made great contributions to the fight against the epidemic by preventing infection, reducing the rate of severe illness, and curbing the spread of the epidemic.

The relatively short research and development cycle of mRNA vaccines enables the rapid development of new vaccine candidates to cope with viral mutations. Through the dual mechanism of humoral immunity and T-cell immunity, the vaccines are highly immunogenic and effective, and have a simple production process, which makes them easy to efficiently develop and produce in a large scale, so as to provide a quick and rapid global supply for the fight against the pandemic similar to COVID-19.

According to the latest news, there has been a breakthrough in the research of circular RNA (circRNA)-related drugs, in addition to mRNA. Orna Therapeutics has developed in-vivo cell therapy products using circRNA, and in a research report presented at the annual meeting of the American Society of Gene and Cell Therapy (ASGCT) in 2022, it has been demonstrated that these products also have great potential in other fields, such as tumor therapy.

RNA shines in the field of research and development of drugs such as vaccines, but the removal of some impurities in the actual production process requires further study, among which double-stranded RNA (dsRNA) impurities can cause strong immunogenicity (Goubau et al., 2014; Kato et al., 2006; Mu et al., 2018). Therefore, there is an urgent need to develop a method for effectively reducing double-stranded RNA impurities.

### SUMMARY OF THE INVENTION

The present application provides an RNA polymerase variant, a preparation method therefor, and the use thereof in RNA synthesis. In a first aspect of the present application, the present application provides an RNA polymerase variant, which has an amino acid sequence comprising, relative to SEQ ID NO: 1, at least one, at least two, at least three, at least four, at least five or at least six amino acid mutations at positions selected from D130, N171, K172, R173, Y178, R298, Y385, K387, D388 or F880, wherein the mutation type is selected from a deletion or substitution, and the amino acid sequence of the variant has at least 95%, 96%, 97%, 98% or 99% sequence identity to SEQ ID NO: 1. In some embodiments, the variant comprises one, two, three, four, five or six amino acid mutations at positions selected from D130, N171, K172, R173, Y178, R298, Y385, K387, D388 or F880; the mutation type is selected from a deletion or substitution. In some embodiments, the mutation type is a deletion (denoted by DEL, e.g., DEL5, indicating an amino acid deletion at site 5). In some embodiments, the mutation type is a substitution (e.g., K5A, indicating a mutation of lysine at site 5 into alanine). In some embodiments, the mutation type comprises deletions and substitutions, i.e., deletions occurring at some positions and substitutions occurring at some other positions.

In some embodiments, the RNA polymerase variant provided by the present application has an amino acid sequence having, relative to SEQ ID NO: 1, one amino acid mutation, the site of the amino acid mutation is selected from N171, K172, R173, Y178, R298, Y385, K387, D388 or F880, and the mutation type is a substitution or deletion. In some embodiments, the amino acid sequence of the variant has, relative to SEQ ID NO: 1, one amino acid mutation, and the mutation is selected from:

(1) a substitution at position N171, wherein the amino acid for the substitution is G; or
(2) a substitution or deletion at position K172, wherein an amino acid for the substitution may be selected from A, G, E, D, H, Y, S, W, P, I, M, V, F, T, C, N, L; or
(3) a substitution or deletion at position R173, wherein an amino acid for the substitution may be selected from A, C, G, E, D, H, Y, S, W, P, N, Q; or
(4) a substitution at position Y178, wherein an amino acid for the substitution is H; or
(5) a substitution at position R298, wherein an amino acid for the substitution is A; or

(6) a substitution at position Y385, wherein an amino acid for the substitution may be selected from A, E, D; or

(7) a substitution at position K387, wherein an amino acid for the substitution may be selected from A, Y, S, Q; or

(8) a substitution at position D388, wherein an amino acid for the substitution may be selected from A, G, L; or

(9) a substitution at position F880, wherein an amino acid for the substitution may be selected from A, G, W.

In some embodiments, the RNA polymerase variant provided by the present application has an amino acid sequence comprising, relative to SEQ ID NO: 1, two, three, four or five amino acid mutations at positions selected from D130, K172, R173, Y178, R298, Y385, K387, D388 or F880, and the amino acid sequence of the variant has at least 95%, 96%, 97%, 98%, 99% or 99.5% sequence identity to SEQ ID NO: 1. In some embodiments, the variant comprises: (1) a mutation at position K172, and also one, two, three or four amino acid mutations at positions selected from D130, R173, Y178, R298, Y385, K387, D388 or F880, wherein the mutation type is a substitution or deletion; or (2) a mutation at position R173, and also one, two or three amino acid mutations at positions selected from D130, Y178, R298, K387 or D388, wherein the mutation type is a substitution or deletion; or (3) two or three amino acid mutations at positions selected from R298, Y385, K387 or D388, wherein the mutation type is a substitution or deletion. In some embodiments, the amino acid sequence of the variant has, relative to SEQ ID NO: 1, a mutation site combination selected from any one of: K172+R173, D130+K172, K172+K387, K172+F880, K172+D388, K172+R298, D130+R173, R173+Y178, R173+D388, R173+R298, K387+R298, Y385+R298, D388+R298, Y385+K387, Y385+D388, K387+D388, K172+R173+Y385, K172+R173+D388, K172+R173+K387, K172+R173+F880, K172+R173+Y178, D130+K172+R173, D130+K172+Y178, D130+K172+K387, D130+R173+D388, K172+Y178+D388, D130+K172+D388, K172+K387+R298, Y385+K387+D388, K172+R173+Y385+F880, K172+R173+D388+F880, K172+R173+Y178+D388, D130+K172+R173+D388, D130+K172+R173+Y178, D130+R173+Y178+K387, D130+K172+Y178+D388, D130+K172+R173+Y178+D388. In some embodiments, further, the mutation type at position D130 in the variant is D130E. In some embodiments, further, the mutation type at position K172 in the variant may be selected from DEL172, K172A or K172G. In some embodiments, further, the mutation type at position R173 in the variant may be selected from DEL173, R173A, R173G or R173C. In some embodiments, further, the mutation type at position Y178 in the variant may be selected from Y178H or Y178P. In some embodiments, further, the mutation type at position R298 in the variant is R298A. In some embodiments, further, the mutation type at position Y385 in the variant is Y385A. In some embodiments, further, the mutation type at position K387 in the variant may be selected from K387S, K387Y or K387G. In some embodiments, further, the mutation type at position D388 in the variant may be selected from D388Y, D388A or D388G. In some embodiments, further, the mutation type at position F880 in the variant may be selected from F880A or F880Y.

In some embodiments, the variant comprises a mutation at position K172, and also comprises one, two, three or four amino acid mutations at positions selected from D130, R173, Y178, R298, Y385, K387, D388 or F880, the mutation type is a substitution or deletion, and the amino acid sequence of the variant has at least 95%, 96%, 97%, 98%, 99% or 99.5% sequence identity to SEQ ID NO: 1. In some embodiments, the variant comprises a mutation combination selected from any one of: K172+R173, D130+K172, K172+K387, K172+F880, K172+D388, K172+R298, K172+R173+Y385, K172+R173+D388, K172+R173+K387, K172+R173+F880, K172+R173+Y178, D130+K172+R173, D130+K172+Y178, D130+K172+K387, K172+Y178+D388, D130+K172+D388, K172+K387+R298, K172+R173+Y385+F880, K172+R173+D388+F880, K172+R173+Y178+D388, D130+K172+R173+D388, D130+K172+R173+Y178, D130+K172+Y178+D388, D130+K172+R173+Y178+D388. In some embodiments, further, the mutation type at position D130 in the variant is D130E. In some embodiments, further, the mutation type at position K172 in the variant may be selected from DEL172, K172A or K172G. In some embodiments, further, the mutation type at position R173 in the variant may be selected from DEL173, R173A, R173G or R173C. In some embodiments, further, the mutation type at position Y178 in the variant is Y178H. Further, the mutation type at position R298 in the variant is R298A. In some embodiments, further, the mutation type at position Y385 in the variant is Y385A. In some embodiments, further, the mutation type at position K387 in the variant may be selected from K387S, K387Y or K387G. In some embodiments, further, the mutation type at position D388 in the variant may be selected from D388Y, D388A or D388G. In some embodiments, further, the mutation type at position F880 in the variant may be selected from F880A or F880Y.

In some embodiments, the variant comprises a mutation at position R173, and also comprises one, two or three amino acid mutations at positions selected from D130, Y178, R298, K387, D388 or F880, the mutation type is a substitution or deletion, and the amino acid sequence of the variant has at least 95%, 96%, 97%, 98%, 99% or 99.5% sequence identity to SEQ ID NO: 1. In some embodiments, the variant comprises a mutation combination selected from any one of: D130+R173, R173+Y178, R173+D388, R173+R298, D130+R173+D388, D130+R173+Y178+K387. In some embodiments, further, the mutation type at position D130 in the variant is D130E. In some embodiments, further, the mutation type at position R173 in the variant may be selected from DEL173, R173A, R173G or R173C. In some embodiments, further, the mutation type at position Y178 in the variant is Y178H or Y178P. In some embodiments, further, the mutation type at position R298 in the variant is R298A. In some embodiments, further, the mutation type at position K387 in the variant is K387Y. In some embodiments, further, the mutation type at position D388 in the variant may be selected from D388Y or D388G.

In some embodiments, the variant comprises any two or three amino acid mutations at positions selected from R298, Y385, K387 or D388, the mutation type is a substitution or deletion, and the amino acid sequence of the variant has at least 95%, 96%, 97%, 98%, 99% or 99.5% sequence identity to SEQ ID NO: 1. In some embodiments, the variant comprises a mutation combination selected from any one of: K387+R298, Y385+R298, D388+R298, Y385+K387, Y385+D388, K387+D388, Y385+K387+D388. In some embodiments, further, the mutation type at position R298 in the variant is R298A. In some embodiments, further, the substitution at position Y385 in the variant is Y385A. In some embodiments, further, the substitution at position K387 in the variant may be selected from K387S or K387Y. In some embodiments, further, the substitution at position D388 in the variant may be selected from D388A or D388G.

In some embodiments, the amino acid sequence of the variant of the present application has at least 99.0%, at least 99.1%, at least 99.2%, at least 99.3%, at least 99.4%, at least 99.5%, at least 99.6%, at least 99.7%, at least 99.8%, at least 99.9% or more sequence identity to the amino acid sequence selected from any one of amino acid sequences set forth in SEQ ID NOs: 2-141. In some embodiments, the amino acid sequence of the variant of the present application is selected from SEQ ID NOs: 2-141.

In a second aspect, the present application provides a method for preparing an RNA polymerase variant, which method comprises generating at least one RNA polymerase variant of the present application in a host cell. In some embodiments, the host cell contains an expression vector carrying a nucleotide sequence corresponding to the RNA polymerase variant of the present application. In some embodiments, in the nucleotide sequence, various modifications may be present in the coding region, as long as the amino acid sequence of the variants of the present application does not vary with the degeneracy of codons or does not vary with preferred codons in an organism expressing the variant. In some embodiments, the nucleotide sequence is selected from SEQ ID NOs: 143-282.

In a third aspect, the present application provides a method for reducing the generation of dsRNA impurities during the preparation of RNA by in-vitro transcription, which method comprises bringing a DNA template into contact with one or more RNA polymerase variants of the present application, and performing incubation in an in-vitro transcription system.

In a fourth aspect, the present application provides a method for increasing the integrity of an RNA product in in-vitro transcription, which method comprises bringing a DNA template into contact with one or more RNA polymerase variants of the present application, and performing incubation in an in-vitro transcription system.

In a fifth aspect, the present application provides a method for generating RNA, which method comprises under conditions resulting in the generation of an RNA product in transcription, bringing a DNA template into contact with one or more RNA polymerase variants of the present application, and performing incubation in an in-vitro transcription system.

In some embodiments, the RNA prepared using the method of the present application may be a coding RNA or a non-coding RNA, including, but not limited to mRNA, siRNA, gRNA, saRNA, dsRNA, ssRNA, miRNA, piRNA, shRNA, etc. In some embodiments, the RNA product is mRNA. In some embodiments, the RNA product is saRNA.

In some embodiments, the length of the DNA template may be selected from 1000-13000 bp. In some embodiments, the length of the DNA template may be selected from 8000-13000 bp. In some embodiments, the length of the DNA template may be selected from 10000-13000 bp.

In some embodiments, the RNA generated using the method of the present application has, upon purification, a reduction in residual dsRNA impurities by at least 50%, at least 55%, at least 60%, at least 65%, at least 70%, at least 75%, at least 80%, at least 85%, at least 90%, at least 95% or more compared with that using wild-type RNA polymerase (SEQ ID NO: 1). In some embodiments, the RNA generated using the method of the present application has, upon purification, a dsRNA impurity percentage (residual dsRNA impurities/total RNA) of less than 0.04%, less than 0.03%, less than 0.02%, less than 0.01%, less than 0.005%, less than 0.004%, less than 0.003%, less than 0.002%, less than 0.001%, less than 0.0005%, less than 0.0003% or less than 0.0001%.

In some embodiments, the mRNA generated using the method of the present application has, upon purification, an increase in the integrity of mRNA product by at least about 1%, at least about 2%, about 3%, about 4%, about 5%, about 6%, about 7%, about 8%, about 9%, about 10%, about 11%, about 12%, about 13%, about 14% or about 15% compared with that using wild-type T7 RNA polymerase (SEQ ID NO: 1).

In some embodiments, the saRNA generated using the method of the present application has, upon purification, an increase in the integrity of saRNA product by at least about 3%, about 5%, about 10%, about 12%, about 14%, about 15%, about 16%, about 17%, about 18%, about 19%, about 20%, about 21%, about 22%, about 23%, about 24%, about 25%, about 26%, about 27%, about 28%, about 29% or about 30% compared with that using wild-type T7 RNA polymerase.

In a sixth aspect, the present application provides a method for generating capped RNA by in-vitro transcription, which method comprises incubating a DNA template with one or more RNA polymerase variants of the present application and a cap analog in an in-vitro transcription reaction system.

In some embodiments, the cap analog is a dinucleotide cap, a trinucleotide cap or a tetranucleotide cap. In some embodiments, the cap analog is a trinucleotide cap. In some embodiments, the trinucleotide cap is selected from GAA, GAC, GAG, GAU, GCA, GCC, GCG, GCU, GGA, GGC, GGG, GGU, GUA, GUC, GUG and GUU. In some embodiments,

**EP 4 502 154 A2**

the trinucleotide cap is selected from $m^7GpppApA$, $m^7GpppApC$, $m^7GpppApG$, $m^7GpppApU$, $m^7GpppCpA$, $m^7GpppCpC$, $m^7GpppCpG$, $m^7GpppCpU$, $m^7GpppGpA$, $m^7GpppGpC$, $m^7GpppGpG$, $m^7GpppGpU$, $m^7GpppUpA$, $m^7GpppUpC$, $m^7GpppUpG$, and $m^7GpppUpU$. In some embodiments, the trinucleotide cap is selected from $m^7G_{3'O-Me}pppApA$, $m^7G_{3'OMe}pppApC$, $m^7G_{3'OMe}pppApG$, $m^7G_{3'OMe}pppApU$, $m^7G_{3'OMe}pppCpA$, $m^7G_{3'OMe}pppCpC$, $m^7G_{3'O-Me}pppCpG$, $m^7G_{3'OMe}pppCpU$, $m^7G_{3'OMe}pppGpA$, $m^7G_{3'OMe}pppGpC$, $m^7G_{3'OMe}pppGpG$, $m^7G_{3'OMe}pppGpU$, $m^7G_{3'O-Me}pppUpA$, $m^7G_{3'OMe}pppUpC$, $m^7G_{3'OMe}pppUpG$, and $m^7G_{3'OMe}pppUpU$. In some embodiments, the trinucleotide cap is selected from $m^7G_{3'OMe}pppA_{2'OMe}pA$, $m^7G_{3'OMe}pppA_{2'OMe}pC$, $m^7G_{3'OMe}pppA_{2'OMe}pG$, $m^7G_{3'OMe}pppA_{2'OMe}pU$, $m^7G_{3'OMe}pppC_{2'OMe}pA$, $m^7G_{3'OMe}pppC_{2'OMe}pC$, $m^7G_{3'OMe}pppC_{2'OMe}pG$, $m^7G_{3'OMe}pppC_{2'OMe}pU$, $m^7G_{3'OMe}pppG_{2'O-Me}pA$, $m^7G_{3'OMe}pppG_{2'OMe}pC$, $m^7G_{3'OMe}pppG_{2'OMe}pG$, $m^7G_{3'OMe}pppG_{2'OMe}pU$, $m^7G_{3'OMe}pppU_{2'OMe}pA$, $m^7G_{3'OMe}pp-pU_{2'OMe}pC$, $m^7G_{3'OMe}pppU_{2'OMe}pG$, and $m^7G_{3'OMe}pppU_{2'OMe}pU$. In some embodiments, the trinucleotide cap is selected from $m^7GpppA_{2'OMe}pA$, $m^7GpppA_{2'OMe}pC$, $m^7GpppA_{2'OMe}pG$, $m^7GpppA_{2'OMe}pU$, $m^7GpppC_{2'OMe}pA$, $m^7GpppC_{2'OMe}pC$, $m^7GpppC_{2'OMe}pG$, $m^7GpppC_{2'OMe}pU$, $m^7GpppG_{2'OMe}pA$, $m^7GpppG_{2'OMe}pC$, $m^7GpppG_{2'OMe}pG$, $m^7GpppG_{2'OMe}pU$, $m^7GpppU_{2'OMe}pA$, $m^7GpppU_{2'OMe}pC$, $m^7GpppU_{2'OMe}pG$, and $m^7GpppU_{2'OMe}pU$. In some embodiments, the trinucleotide cap is preferably m7GpppA2'OMepG.

In some embodiment, the in-vitro transcriptional capping reaction using the polymerase variant of the present application has an increase in the capping rate of mRNA product to 90%, 91%, 92%, 93%, 94%, 95%, 96%, 97%, 98%, 99% or 100% compared with that using wild-type T7 RNA polymerase (SEQ ID NO: 1). In some embodiments, the increase in the capping rate of mRNA product may be 100%.

In a seventh aspect, the present application provides a method of performing in-vitro transcription, which method comprises under conditions resulting in the generation of an RNA product in transcription, bringing a DNA template into contact with one or more RNA polymerase variants of the present application, and performing incubation in an in-vitro transcription system. In some embodiments, the method comprises the following steps: 1) providing a DNA template containing a T7 promoter, wherein the T7 promoter is functionally linked to a target nucleotide sequence to be transcribed; 2) bringing one or more RNA polymerase variants of the present application into contact with the DNA template in step 1); 3) incubating the DNA template and the RNA polymerase variant in an in-vitro transcription system.

In some embodiments, the incubation temperature in step 3) is 30°C to 50°C, preferably 37°C. In some embodiments, the incubation time in step 3) is 20 to 240 min, preferably 60 min.

The in-vitro transcription system of the present application comprises a riboside triphosphate and a buffer component.

In some embodiments, the riboside triphosphate may be selected from a modified or unmodified nucleoside triphosphate (including analogs thereof). In some embodiments, the riboside triphosphate may be selected from unmodified ATP, GTP, CTP, UTP. In some embodiments, the riboside triphosphate may be selected from a modified nucleoside triphosphate, including, but not limited to m1A (N1-methyladenosine), m6A (N6-methyladenosine), m5C (5-methylcytidine), 5moU (5-methoxyuridine), $\psi$ (pseudouridine), m1$\psi$ (N1-methyl-pseudouridine), riboside triphosphate with a marker (the marker may be biotin, a fluorescent substance, digoxin, a radioactive element, etc.).

In some embodiments, the in-vitro transcription system further comprises an RNase inhibitor, an inorganic pyrophosphatase, magnesium ions. In some embodiments, the in-vitro transcription system further comprises DEPC-treated water.

In an eighth aspect, the present application provides a composition or a kit, which comprises one or more RNA polymerase variants of the present application.

In one embodiment, the composition or the kit further comprises one or more buffer components. In one embodiment, the composition or the kit further comprises one or more in-vitro transcription system components, wherein the in-vitro transcription component may be selected from a riboside triphosphate, an RNase inhibitor, an inorganic pyrophosphatase, magnesium ions, etc. In one embodiment, the in-vitro transcription system component may be selected from a commercially available mRNA in-vitro transcription reagent.

In some embodiments, the composition or the kit further comprises a cap analog. In some embodiments, the cap analog is a dinucleotide cap, a trinucleotide cap or a tetranucleotide cap. In some embodiments, the cap analog is a trinucleotide cap. In some embodiments, the trinucleotide cap is selected from GAA, GAC, GAG, GAU, GCA, GCC, GCG, GCU, GGA, GGC, GGG, GGU, GUA, GUC, GUG and GUU. In some embodiments, the trinucleotide cap is preferably m7GpppA2'OMepG.

In a ninth aspect, the present application provides a composition, which comprises RNA and a pharmaceutically acceptable excipient, wherein the RNA is generated by the in-vitro transcription method of the present application. In some embodiments, the RNA product is not chemically modified. In some embodiments, the RNA product is chemically modified.

Other embodiments:

5

1. An RNA polymerase variant, having an amino acid sequence comprising, relative to SEQ ID NO: 1, one, two, three, four or five amino acid mutations at positions selected from D130, N171, K172, R173, Y178, R298, Y385, K387, D388 or F880, wherein the mutation type is a substitution or deletion, and the amino acid sequence of the variant has at least 97%, at least 98% or at least 99% sequence identity to SEQ ID NO: 1.

2. The variant according to item 1, wherein the amino acid sequence of the variant has, relative to SEQ ID NO: 1, one amino acid mutation, and the position of the amino acid mutation is selected from N171, K172, R173, Y178, R298, Y385, K387, D388 or F880.

3. The variant according to item 1, wherein the amino acid sequence of the variant comprises, relative to SEQ ID NO: 1:

  (1) a mutation at position K172, and also any one, two, three or four amino acid mutations at positions selected from D130, R173, Y178, R298, Y385, K387, D388 or F880, wherein the mutation type is a substitution or deletion; or
  (2) a mutation at site K173, and also any one, two or three amino acid mutations at positions selected from D130, Y178, R298, K387 or D388, wherein the mutation type is a substitution or deletion; or
  (3) any two or three amino acid mutations at positions selected from R298, Y385, K387 or D388, wherein the mutation type is a substitution.

4. The variant according to item 3, wherein the variant comprises a mutation combination selected from any one of: K172+R173, D130+K172, K172+K387, K172+F880, K172+D388, K172+R298, D130+R173, R173+Y178, R173+D388, R173+R298, K387+R298, Y385+R298, D388+R298, Y385+K387, Y385+D388, K387+D388, K172+R173+Y385, K172+R173+D388, K172+R173+K387, K172+R173+F880, K172+R173+Y178, D130+K172+R173, D130+K172+Y178, D130+K172+K387, D130+R173+D388, K172+Y178+D388, D130+K172+D388, K172+K387+R298, Y385+K387+D388, K172+R173+Y385+F880, K172+R173+D388+F880, K172+R173+Y178+D388, D130+K172+R173+D388, D130+K172+R173+Y178, D130+R173+Y178+K387, D130+K172+Y178+D388, or D130+K172+R173+Y178+D388.

5. The variant according to item 2, wherein

  1) the mutation type at position N171 is N171G;
  2) the mutation type at position K172 is selected from DEL172, K172A, K172H, K172R, K172Y, K172S, K172E, K172D, K172W, K172F, K172I, K172M, K172V, K172P, K172T, K172C, K172N, K172Q, K172G or K172L;
  3) the mutation type at position R173 is selected from DEL173, R173A, R173H, R173R, R173Y, R173S, R173E, R173D, R173W, R173F, R173I, R173M, R173V, R173P, R173T, R173C, R173N, R173Q, R173G or R173L;
  4) the mutation type at position Y178 is Y178H;
  5) the mutation type at position R298 is R298A;
  6) the mutation type at position Y385 is selected from Y385A, Y385D or Y385E;
  7) the mutation type at position K387 is selected from K387Q, K387Y, K387S or K3 87A;
  8) the mutation type at position D388 is selected from D388A, D388G or D388L; and
  9) the mutation type at position F880 is selected from F880A, F880G or F880W.

6. The variant according to item 3 or item 4, wherein

  1) the mutation type at position D130 is D130E;
  2) the mutation type at position K172 may be selected from DEL172, K172A or K172G;
  3) the mutation type at position R173 may be selected from DEL173, R173A, R173G or R173C;
  4) the mutation type at position Y178 may be selected from Y178H or Y178P;
  5) the mutation type at position R298 is R298A;
  6) the mutation type at position Y385 is Y385A;
  7) the mutation type at position K387 may be selected from K387S, K387Y or K387G;
  8) the mutation type at position D388 may be selected from D388Y, D388A or D388G; and
  9) the mutation type at position F880 may be selected from F880A or F880Y.

7. The variant according to item 1, wherein the amino acid sequence of the variant is set forth in any one of SEQ ID NOs: 2-141.

8. A nucleotide sequence encoding the variant according to any one of items 1-7.

9. A method for preparing an RNA polymerase variant, comprising generating at least one RNA polymerase variant according to any one of items 1-7 in a host cell, wherein the host cell contains an expression vector carrying the nucleotide sequence according to item 8.

10. A method for reducing the generation of dsRNA impurities during the preparation of RNA by in-vitro transcription, comprising bringing a DNA template into contact with the variant according to any one of items 1-7, and performing incubation in an in-vitro transcription system.

11. A method for increasing the integrity of an RNA product in in-vitro transcription, comprising bringing a DNA template into contact with the variant according to any one of items 1-7, and performing incubation in an in-vitro transcription system.

12. Use of at least one variant according to any one of items 1-7 or the method according to items 10-11 in the generation of RNA by in-vitro transcription.

13. A method for generating RNA by in-vitro transcription, comprising bringing a DNA template into contact with at least one variant according to any one of items 1-7, and performing incubation in an in-vitro transcription system.

14. A method for generating capped RNA by in-vitro transcription, comprising bringing a DNA template into contact with at least one variant according to any one of items 1-7 and a cap analog, and performing incubation in an in-vitro transcription reaction system.

15. A composition or kit, comprising at least one RNA polymerase variant according to any one of items 1-7 and at least one buffer component.

16. The composition or kit according to item 15, further comprising a cap analog.

The present application has the following beneficial effects:

The RNA polymerase variants of the present application have high catalytic efficiency, and can reduce the generation of double-stranded RNA impurities when used in mRNA synthesis. Compared with the existing reported mRNA preparation method, the preparation method of the present application has the lowest generation of double-stranded RNA impurities, and is a safe and green mRNA preparation method with a low content of double-stranded RNA impurities. Using the mRNA preparation method of the present application, the possibility of immunogenicity caused by double-stranded RNA impurities can be greatly reduced. In addition, some of the RNA polymerase variants provided by the present application are able of increasing the integrity of RNA product in in-vitro transcription. The variant DEL172+K387S can also improve the utilization efficiency of the cap analog in the co-transcriptional capping reaction, thus saving costs.

**BRIEF DESCRIPTION OF THE DRAWINGS**

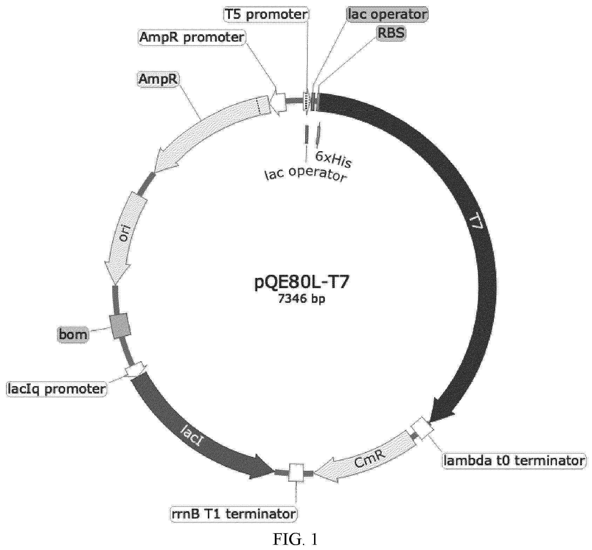

FIG. 1 is a schematic diagram of the construction of recombinant plasmid.

**DETAILED DESCRIPTION OF EMBODIMENTS**

In the examples of the present application, the enzyme activity is defined as follows: the amount of enzyme required to generate 1 $\mu$mol product or convert 1 $\mu$mol substrate in 1 min under specific reaction conditions.

**Example 1 Preparation of RNA polymerase variants**

The DNA sequences (SEQ ID NOs: 142-282) of the RNA polymerase and the variants thereof as shown in Table 1 were synthesized, then amplified by PCR, and then respectively introduced into the BseRI and HindIII cleavage sites of an expression vector pQE-80L to obtain recombinant expression vectors. The constructed vector was introduced into *E. coli* BL21 (DE3) by chemical transformation technology. The transformed *E. coli* BL21 (DE3) was coated on an ampicillin-resistant LB plate, and the plate was placed in an incubator at 37°C overnight. The single colonies growing in the plate were subjected to plasma extraction and sequencing, and finally recombinant engineered bacteria containing the gene of interest were obtained. The E. *coli* recombinant strain with correct sequencing was inoculated into an LB medium for activation culture overnight, then inoculated into a fermentation broth (LB medium) at 1% to 5% V/V, and cultured until the OD 600 value was 0.6 to 0.8. IPTG with a final concentration of 0.5 mol/L was added for continuing culture for 4-6 h. The strain was collected by centrifugation at 5°C at 12000 rpm. The collected strain was washed with a 0.2 M PBS buffer with a pH value of 7.0 to obtain bacteria. After ultrasonic disruption, purification was performed by affinity chromatography to obtain an RNA polymerase stock solution.

WT is a wild-type T7 RNA polymerase variant having an amino acid sequence:

MNTINIAKNDFSDIELAAIPFNTLADHYGERLAREQLALEHESYEMGEARFRKMF

ERQLKAGEVADNAAAKPLITTLLPKMIARINDWFEEVKAKRGKRPTAFQFLQEIKPEAV

AYITIKTTLACLTSADNTTVQAVASAIGRAIEDEARFGRIRDLEAKHFKKNVEEQLNKRV

---

7

GHVYKKAFMQVVEADMLSKGLLGGEAWSSWHKEDSIHVGVRCIEMLIESTGMVSLHR
QNAGVVGQDSETIELAPEYAEAIATRAGALAGISPMFQPCVVPPKPWTGITGGGYWANG
RRPLALVRTHSKKALMRYEDVYMPEVYKAINIAQNTAWKINKKVLAVANVITKWKHC
PVEDIPAIEREELPMKPEDIDMNPEALTAWKRAAAAVYRKDKARKSRRISLEFMLEQAN
KFANHKAIWFPYNMDWRGRVYAVSMFNPQGNDMTKGLLTLAKGKPIGKEGYYWLKI
HGANCAGVDKVPFPERIKFIEENHENIMACAKSPLENTWWAEQDSPFCFLAFCFEYAGV
QHHGLSYNCSLPLAFDGSCSGIQHFSAMLRDEVGGRAVNLLPSETVQDIYGIVAKKVNEI
LQADAINGTDNEVVTVTDENTGEISEKVKLGTKALAGQWLAYGVTRSVTKRSVMTLA
YGSKEFGFRQQVLEDTIQPAIDSGKGLMFTQPNQAAGYMAKLIWESVSVTVVAAVEAM
NWLKSAAKLLAAEVKDKKTGEILRKRCAVHWVTPDGFPVWQEYKKPIQTRLNLMFLG
QFRLQPTINTNKDSEIDAHKQESGIAPNFVHSQDGSHLRKTVVWAHEKYGIESFALIHDS
FGTIPADAANLFKAVRETMVDTYESCDVLADFYDQFADQLHESQLDKMPALPAKGNLN
LRDILESDFAFA (SEQ ID NO: 1)

The RNA polymerase variants and the mutation sites thereof are as shown in Table 1:

Table 1: Mutation sites of RNA polymerase variants and corresponding amino acid sequence numbers

| No. | Mutation site (relative to SEQ ID NO: 1) | No. | Mutation site (relative to SEQ ID NO: 1) |
|---|---|---|---|
| 1 | WT | | |
| 2 | K172A | 72 | D130E+R173A |
| 3 | K172G | 73 | D130E+K172A+Y178H+D388Y |
| 4 | DEL172 | 74 | D130E+K172A+R173G+Y178H |
| 5 | DEL173 | 75 | K172A+Y178H+D388Y |
| 6 | R173C | 76 | D130E+K172G+D388Y |
| 7 | R173G | 77 | D130E+K172A+R173C+Y178H+D 388Y |
| 8 | K172A+R173A | 78 | D130E+K172G+R173C+Y178H+D 388G |
| 9 | K172G+R173A | 79 | D130E+K172A+R173G+D388Y |
| 10 | K172A+R173G | 80 | K172A+R173C+D388Y |
| 11 | K172G+R173G | 81 | K172A+R173A+D388Y |
| 12 | DEL172-173 | 82 | DEL172+K387G |
| 13 | DEL172-173+Y385A | 83 | D130E+R173G+Y178P+K387Y |
| 14 | DEL172-173+D388A | 84 | DEL172-173+K387G |
| 15 | DEL172-173+D388G | 85 | DEL172-173+K387Y |
| 16 | DEL172-173+Y385A+F880Y | 86 | DEL172+R173A+K387S |
| 17 | DEL172-173+D388G+F880Y | 87 | DEL172+R173 G+K387S |
| 18 | Y178H | 88 | K172A+DEL173 |
| 19 | Y385A | 89 | K172G+DEL173 |
| 20 | Y385D | 90 | DEL172+R298A |
| 21 | F880A | 91 | DEL173+R298A |

(continued)

| No. | Mutation site (relative to SEQ ID NO: 1) | No. | Mutation site (relative to SEQ ID NO: 1) |
|---|---|---|---|
| 22 | F880G | 92 | K387S+R298A |
| 23 | F880W | 93 | K387Y+R298A |
| 24 | D388A | 94 | Y385A+R298A |
| 25 | D388G | 95 | D388A+R298A |
| 26 | N171G | 96 | DEL172+K387S+R298A |
| 27 | K172E | 97 | Y385A+K387Y |
| 28 | K172D | 98 | Y385A+K387S |
| 29 | R173E | 99 | Y385A+D388G |
| 30 | R173D | 100 | K387Y+D388A |
| 31 | R173A | 101 | K387Y+D388G |
| 32 | K172A+R173G+Y385A | 102 | K387S+D388A |
| 33 | K172A+R173G+D388Y | 103 | Y385A+K387Y+D388G |
| 34 | K172A+R173G+F880Y | 104 | Y385A+K387S+D388G |
| 35 | K172G+R173G+Y385A | 105 | Y385A+K387Y+D388A |
| 36 | K172G+R173G+D388Y | 106 | Y385A+K387S+D388A |
| 37 | K172G+R173G+F880Y | 107 | K172H |
| 38 | DEL172-173+F880Y | 108 | K172Y |
| 39 | DEL172-173+Y178H | 109 | K172S |
| 40 | DEL172-173+D130E | 110 | K172W |
| 41 | DEL172+R173A | 111 | K172F |
| 42 | DEL172+R173C | 112 | K172I |
| 43 | DEL172+R173G | 113 | K172M |
| 44 | DEL172+D130E | 114 | K172V |
| 45 | DEL172+K387S | 115 | K172P |
| 46 | DEL172+K387Y | 116 | K172T |
| 47 | DEL172+F880A | 117 | K172C |
| 48 | R298A | 118 | K172N |
| 49 | Y385E | 119 | K172L |
| 50 | K387Q | 120 | R173H |
| 51 | K387Y | 121 | R173Y |
| 52 | K387S | 122 | R173S |
| 53 | D388L | 123 | R173W |
| 54 | D130E+K172A+R173G+Y178H+D3 88G | 124 | R173P |
| 55 | D130E+R173G | 125 | R173N |
| 56 | D130E+K172G+Y178H+D388G | 126 | R173Q |
| 57 | K172A+R173C+Y178H+D388G | 127 | K172A+R173A+K387Y |
| 58 | D130E+K172G+R173C+D388Y | 128 | K172A+R173G+K387Y |
| 59 | D130E+K172G+R173G+Y178H | 129 | K172A+DEL173+K387Y |
| 60 | R173A+Y178H | 130 | K172G+R173A+K387Y |

(continued)

| No. | Mutation site (relative to SEQ ID NO: 1) | No. | Mutation site (relative to SEQ ID NO: 1) |
|---|---|---|---|
| 61 | D130E+K172A+Y178H | 131 | K172G+R173G+K387Y |
| 62 | D130E+K172A+R173A | 132 | K172G+DEL173+K387Y |
| 63 | D130E+K172G+R173G+D388G | 133 | K172A+R173A+K387S |
| 64 | D130E+K172G+R173C+Y178H+D3 88Y | 134 | K172A+R173G+K387S |
| 65 | K172G+D388Y | 135 | K172A+DEL173+K387S |
| 66 | D130E+K172A | 136 | K172G+R173A+K387S |
| 67 | D130E+R173A+D388G | 137 | K172G+R173G+K387S |
| 68 | K172A+D388Y | 138 | K172G+DEL173+K387S |
| 69 | D130E+K172A+R173C+D388G | 139 | D130E+DEL172+K387Y |
| 70 | D130E+R173C | 140 | D130E+DEL172 |
| 71 | R173A+D388Y | 141 | K387A |

**Example 2 dsRNA impurity generation in in-vitro transcription**

2.1 Unmodified NTP

(1) The enzyme stock solution obtained in Example 1 was diluted with a storage buffer (Vazyme, catalog number: DD4101) to an enzyme activity of 300 U/μL. The reaction components (20 μL) according to Table 2 were added into an 8-tube strip, mixed uniformly, and centrifuged. The 8-tube strip was placed on a PCR instrument and the reaction was performed at 37°C for 1 h. Then 36 μL of magnetic beads were added, and the resulting mixture was mixed uniformly and then incubated at room temperature for 2 to 5 min. The mixture was placed on a magnetic rack to purify mRNA (Vazyme, catalog number: N412). After purification, the resulting product was transferred to an RNase-free centrifuge tube to obtain the purified mRNA.
(2) The content of dsRNA impurities was tested using a dsRNA assay kit (Vazyme, catalog number: DD3509).

Table 2: Reaction system ratio

| Component | Concentration | Volume (μL) |
|---|---|---|
| ATP | 100 mM | 2 |
| GTP | 100 mM | 2 |
| CTP | 100 mM | 2 |
| UTP | 100 mM | 2 |
| Murine RNase Inhibitor (Vazyme, catalog number: DD4102) | 40 U/μL | 1 |
| Pyrophosphatase, Inorganic (Vazyme, catalog number: DD4103) | 0.1 U/μL | 1 |
| Template DNA (SEQ ID NO: 283) | \ | 1 μg |
| 10×Transcription buffer (Vazyme, catalog number: DD4101R) | \ | 2 |
| T7 RNA polymerase | 300 U/μL | 1 |
| ddH$_2$O | \ | Making up to 20 μL |

The dsRNA assay results are as shown in Table 3. Compared with the WT group, most of the polymerase variants in Example 1 can effectively reduce the generation of dsRNA impurities during in-vitro transcription, wherein the variants in the DEL172-173+Y385A, DEL172-173+D388A, and DEL172-173+D388G groups can reduce the residual dsRNA by 99%.

Table 3: dsRNA residue

| Amino acid sequence number | Variant (relative to SEQ ID NO: 1) | dsRNA residue (%) (relative to WT) |
|---|---|---|
| 1 | WT | \ |
| 2 | K172A | 49.90 |
| 3 | K172G | 27.93 |
| 4 | Del172 | 32.42 |
| 5 | Del173 | 27.72 |
| 6 | R173C | 40.62 |
| 7 | R173G | 23.80 |
| 8 | K172A+R173A | 18.35 |
| 9 | K172G+R173A | 8.04 |
| 10 | K172A+R173G | 7.42 |
| 11 | K172G+R173G | 8.05 |
| 12 | DEL172-173 | 3.12 |
| 13 | DEL172-173+Y385A | 0.62 |
| 14 | DEL172-173+D388A | 0.62 |
| 15 | DEL172-173+D388G | 0.62 |
| 16 | DEL172-173+Y385A+F880Y | 3.30 |
| 17 | DEL172-173+D388G+F880Y | 2.06 |
| 18 | Y178H | 19.18 |
| 19 | Y385A | 45.77 |
| 20 | Y385D | 35.46 |
| 21 | F880A | 2.06 |
| 22 | F880G | 4.54 |
| 23 | F880W | 6.19 |
| 24 | D388A | 116.08 |
| 25 | D388G | 130.72 |
| 26 | N171G | 62.16 |
| 27 | K172E | 12.74 |
| 28 | K172D | 9.90 |
| 29 | R173E | 12.24 |
| 30 | R173D | 3.24 |
| 31 | R173A | 26.60 |
| 32 | K172A+R173G+Y385A | 8.31 |
| 33 | K172A+R173G+D388Y | 8.85 |
| 34 | K172A+R173G+F880Y | 20.00 |
| 35 | K172G+R173G+Y385A | 7.91 |
| 36 | K172G+R173G+D388Y | 5.49 |
| 37 | K172G+R173G+F880Y | 6.56 |
| 38 | DEL172-173+F880Y | 3.50 |
| 39 | DEL172-173+Y178H | 4.24 |

(continued)

| Amino acid sequence number | Variant (relative to SEQ ID NO: 1) | dsRNA residue (%) (relative to WT) |
|---|---|---|
| 40 | DEL172-173+D130E | 3.49 |
| 41 | DEL172+R173A | 28.25 |
| 42 | DEL172+R173C | 24.51 |
| 43 | DEL172+R173G | 19.34 |
| 44 | DEL172+D130E | 24.27 |
| 45 | DEL172+K387S | 11.67 |
| 46 | DEL172+K387Y | 15.11 |
| 47 | DEL172+F880A | 3.23 |
| 48 | R298A | 32.36 |
| 49 | Y385E | 45.45 |
| 50 | K387Q | 49.26 |
| 51 | K387Y | 87.72 |
| 52 | K387S | 61.35 |
| 53 | D388L | 84.03 |
| 54 | D130E+K172A+R173G+Y178H+ D388G | 21.43 |
| 55 | D130E+R173G | 31.82 |
| 56 | D130E+K172G+Y178H+D388G | 62.14 |
| 57 | K172A+R173C+Y178H+D388G | 70.78 |
| 58 | D130E+K172G+R173C+D388Y | 16.84 |
| 59 | D130E+K172G+R173G+Y178H | 4.47 |
| 60 | R173A+Y178H | 53.14 |
| 61 | D130E+K172A+Y178H | 68.49 |
| 62 | D130E+K172A+R173A | 30.45 |
| 63 | D130E+K172G+R173G+D388G | 4.17 |
| 64 | D130E+K172G+R173C+Y178H+ D388Y | 17.21 |
| 65 | K172G+D388Y | 15.62 |
| 66 | D130E+K172A | 49.11 |
| 67 | D130E+R173A+D388G | 22.96 |
| 68 | K172A+D388Y | 27.80 |
| 69 | D130E+K172A+R173C+D388G | 49.60 |
| 70 | D130E+R173C | 76.39 |
| 71 | R173A+D388Y | 22.79 |
| 72 | D130E+R173A | 30.85 |
| 73 | D130E+K172A+Y178H+D3 88Y | 30.65 |
| 74 | D130E+K172A+R173G+Y178H | 12.01 |
| 75 | K172A+Y178H+D388Y | 56.52 |
| 76 | D130E+K172G+D388Y | 49.22 |
| 77 | D130E+K172A+R173C+Y178H+ D388Y | 23.64 |
| 78 | D130E+K172G+R173C+Y178H+ D388G | 42.53 |

(continued)

| Amino acid sequence number | Variant (relative to SEQ ID NO: 1) | dsRNA residue (%) (relative to WT) |
|---|---|---|
| 79 | D130E+K172A+R173G+D388Y | 20.94 |
| 80 | K172A+R173C+D388Y | 37.36 |
| 81 | K172A+R173A+D388Y | 25.74 |
| 82 | DEL172+K387G | 21.74 |
| 83 | D130E+R173G+Y178P+K387Y | 20.61 |
| 84 | DEL172-173+K387G | 2.29 |
| 85 | DEL172-173+K387Y | 2.51 |
| 86 | DEL172+R173A+K387S | 18.21 |
| 87 | Del172+R173G+K387S | 4.99 |
| 88 | K172A+DEL173 | 26.39 |
| 89 | K172G+DEL173 | 17.68 |
| 90 | DEL172+R298A | 11.61 |
| 91 | DEL173+R298A | 9.90 |
| 92 | K387S+R298A | 61.35 |
| 93 | K387Y+R298A | 82.64 |
| 94 | Y385A+R298A | 10.17 |
| 95 | D388A+R298A | 33.28 |
| 96 | DEL172+K387S+R298A | 19.38 |
| 97 | Y385A+K387Y | 60.61 |
| 98 | Y385A+K387S | 18.99 |
| 99 | Y385A+D388G | 68.26 |
| 100 | K387Y+D388A | 50.63 |
| 101 | K387Y+D388G | 78.74 |
| 102 | K387S+D388A | 64.31 |
| 103 | Y385A+K387Y+D388G | 56.34 |
| 104 | Y385A+K387S+D388G | 41.84 |
| 105 | Y385A+K387Y+D388A | 26.14 |
| 106 | Y385A+K387S+D388A | 19.14 |
| 107 | K172H | 61.73 |
| 108 | K172Y | 40.24 |
| 109 | K172S | 36.83 |
| 110 | K172W | 25.87 |
| 111 | K172F | 28.33 |
| 112 | K172I | 30.72 |
| 113 | K172M | 76.63 |
| 114 | K172V | 27.74 |
| 115 | K172P | 7.83 |
| 116 | K172T | 34.48 |
| 117 | K172C | 34.31 |

(continued)

| Amino acid sequence number | Variant (relative to SEQ ID NO: 1) | dsRNA residue (%) (relative to WT) |
|---|---|---|
| 118 | K172N | 32.00 |
| 119 | K172L | 56.82 |
| 120 | R173H | 47.28 |
| 121 | R173Y | 41.32 |
| 122 | R173S | 22.32 |
| 123 | R173W | 34.31 |
| 124 | R173P | 9.03 |
| 125 | R173N | 23.23 |
| 126 | R173Q | 45.45 |
| 127 | K172A+R173A+K387Y | 8.02 |
| 128 | K172A+R173G+K387Y | 7.17 |
| 129 | K172A+Del173+K387Y | 8.14 |
| 130 | K172G+R173A+K387Y | 7.63 |
| 131 | K172G+R173G+K387Y | 5.41 |
| 132 | K172G+DEL173+K387Y | 5.70 |
| 133 | K172A+R173A+K387S | 7.54 |
| 134 | K172A+R173G+K387S | 3.98 |
| 135 | K172A+DEL173+K387S | 10.48 |
| 136 | K172G+R173A+K387S | 4.91 |
| 137 | K172G+R173G+K387S | 2.67 |
| 138 | K172G+DEL173+K387S | 5.10 |
| 139 | D130E+DEL172+K387Y | 27.78 |
| 140 | D130E+DEL172 | 24.27 |
| 141 | K387A | 13.30 |
| The dsRNA residue (%) refers to the ratio of the residual dsRNA in the variant group to the residual dsRNA in the WT group. | | |

2.2 Modified NTP (m1ψ)

(1) The enzyme stock solutions of RNA polymerase variants DEL172-173+Y385A, DEL172-173+D388A, and DEL172-173+D388G were each diluted with a storage buffer (Vazyme, catalog number: DD4101) to an enzyme activity of 300 U/μL. The reaction components (20 μL) according to Table 4 were added into an 8-tube strip, mixed uniformly, and centrifuged. The 8-tube strip was placed on a PCR instrument and the reaction was performed at 37°C for 1 h. Then 36 μL of magnetic beads were added, and the resulting mixture was mixed uniformly and incubated at room temperature for 2 to 5 min. The mixture was placed on a magnetic rack to purify mRNA (Vazyme, catalog number: N412). After purification, the resulting product was transferred to an RNase-free centrifuge tube to obtain the purified mRNA.
(2) The content of dsRNA impurities was tested using a dsRNA assay kit (Vazyme, catalog number: DD3509).

Table 4: Reaction system ratio

| Component | Concentration | Volume (μL) |
|---|---|---|
| ATP | 100 mM | 2 |
| GTP | 100 mM | 2 |

(continued)

| Component | Concentration | Volume (μL) |
|---|---|---|
| CTP | 100 mM | 2 |
| m1ψ | 100 mM | 2 |
| Murine RNase Inhibitor (Vazyme, catalog number: DD4102) | 40 U/μL | 1 |
| Pyrophosphatase, Inorganic (Vazyme, catalog number: DD4103) | 0.1 U/μL | 1 |
| Template DNA (SEQ ID NO: 283) | \ | 1 μg |
| 10×Transcription buffer (Vazyme, catalog number: DD4101R) | \ | 2 |
| T7 RNA polymerase | 300 U/μL | 1 |
| ddH₂O | \ | Making up to 20 μL |

The dsRNA assay results show that the in-vitro transcription reactions involving the RNA polymerase variants DEL172-173+D388A and DEL172-173+D388G can reduce the residual dsRNA percentage (residual dsRNA/total RNA) to 0.0003%; and the in-vitro transcription reaction involving the polymerase variant DEL172-173+Y385A can reduce the residual dsRNA percentage to 0.0001%.

**Example 3 Alignment of integrity of RNA products in in-vitro transcription**

3.1 Unmodified NTP (template of SEQ ID NO: 283)

(1) The purified mRNA was obtained with reference to step (1) of point 2.1 in Example 2.
(2) Capillary electrophoresis was performed using a Qsep400 automated nucleic acid analyzer to detect the integrity of the mRNA (intact RNA product/total RNA).

The detection results are as shown in Table 5-1 and Table 5-2.

3.2 Modified NTP (m1ψ, template of SEQ ID NO: 283)

(1) The purified mRNA was obtained with reference to step (1) of point 2.2 in Example 2.
(2) Capillary electrophoresis was performed using a Qsep400 automated nucleic acid analyzer to detect the integrity of the mRNA.

The detection results are as shown in Table 5-1 and Table 5-2.

Table 5-1: Integrity of mRNA products

| Sequence No. | Mutation site (relative to SEQ ID NO: 1) | Integrity (unmodified) | Integrity (m1ψ) |
|---|---|---|---|
| 1 | WT | 83% | 83% |
| 18 | Y178H | 89% | 88% |
| 19 | Y385A | 90.5% | 89.1% |
| 49 | Y385E | 87.70% | 87.40% |
| 50 | K387Q | 88.30% | 86.80% |
| 51 | K387Y | 88.50% | 90.60% |
| 25 | D388G | 91.0% | 89.0% |
| 53 | D388L | 87% | 85% |

Table 5-2: Integrity of mRNA products

| Sequence No. | Mutation site (relative to SEQ ID NO: 1) | Increase in integrity (%) |
|---|---|---|
| 2 | K172A | 2.00% |
| 3 | K172G | 2.00% |
| 4 | Del 172 | 1.70% |
| 5 | Del 173 | 2.60% |
| 6 | R173C | 3.00% |
| 7 | R173G | -0.10% |
| 8 | K172A+R173A | 2.00% |
| 9 | K172G+R173A | 1.90% |
| 10 | K172A+R173G | 2.80% |
| 11 | K172G+R173G | 1.80% |
| 12 | DEL 172-173 | -3.30% |
| 20 | Y385D | 0.10% |
| 27 | K172E | -0.20% |
| 29 | R173E | 2.30% |
| 30 | R173D | 0.60% |
| 31 | R173A | 0.10% |
| 32 | K172A+R173G+Y385A | -0.10% |
| 34 | K172A+R173G+F880Y | 1.10% |
| 40 | DEL172-173+D130E | 0.10% |
| 41 | DEL172+R173A | 3.50% |
| 42 | DEL172+R173C | 2.10% |
| 43 | DEL 172+R 173 G | 2.20% |
| 44 | DEL172+D130E | 0.10% |
| 45 | DEL172+K387S | 3.60% |
| 52 | K387S | 3.00% |
| 69 | D130E+K172A+R173C+D388G | 1.05% |
| 88 | K172A+DEL173 | 2.400% |
| 89 | K172G+DEL173 | 0.750% |
| 97 | Y385A+K387Y | -0.60% |
| 102 | K387S+D388A | 1.90% |
| 104 | Y385A+K387S+D388G | -0.40% |
| 106 | Y385A+K387S+D388A | -0.35% |
| 112 | K 172I | 0.70% |
| 113 | K172M | 2.00% |
| 117 | K172C | -0.30% |
| 118 | K172N | 1.55% |
| 119 | K172L | 0.000% |
| 120 | R173H | 0.000% |
| 122 | R173S | 2.250% |

(continued)

| Sequence No. | Mutation site (relative to SEQ ID NO: 1) | Increase in integrity (%) |
|---|---|---|
| 123 | R173W | -0.550% |
| 125 | R173N | 1.850% |
| 127 | K172A+R173A+K387Y | 0.700% |
| 128 | K172A+R173G+K387Y | 1.150% |
| 129 | K172A+DEL173+K387Y | -0.300% |
| 130 | K172G+R173A+K387Y | 0.550% |
| 131 | K172G+R173G+K387Y | 2.150% |
| 133 | K172A+R173A+K387S | 1.050% |
| 134 | K172A+R173G+K387S | 1.050% |
| 135 | K172A+DEL173+K387S | 0.450% |
| 136 | K172G+R173A+K387S | -0.250% |
| 137 | K172G+R173G+K387S | 0.200% |
| 138 | K172G+DEL173+K387S | -0.450% |

The increase in integrity (%) refers to the difference between the proportion of intact mRNA (to total RNA product) in the variant group and the proportion of intact mRNA in the WT group.

3.3 saRNA template I (template of SEQ ID NO: 284)

(1) The enzyme stock solution of the RNA polymerase variant K387Y was diluted with a storage buffer (Vazyme, catalog number: DD4101) to an enzyme activity of 300 U/μL. In-vitro transcription was performed according to the reaction system in Table 2 to obtain the purified mRNA.
(2) Capillary electrophoresis was performed using a Qsep400 automated nucleic acid analyzer to detect the integrity of the saRNA product.

The detection results show that the integrity of the transcription product is only 56.4% in the in-vitro transcription reaction using the wild-type T7 RNA polymerase (WT), while the integrity of the saRNA product can be increased to 73.1% using the K387Y variant.

3.4 saRNA template II (> 10000 nt)

(1) The enzyme stock solutions of the RNA polymerase variants K387A and DEL172+K387Y were each diluted with a storage buffer (Vazyme, catalog number: DD4101) to an enzyme activity of 300 U/μL. In-vitro transcription was performed according to the reaction system in Table 6 to obtain the purified mRNA.

Table 6: Reaction system

| Component | Concentration | Volume (μL) |
|---|---|---|
| ATP | 100 mM | 1.5 |
| GTP | 100 mM | 1.5 |
| CTP | 100 mM | 1.5 |
| UTP | 100 mM | 1.5 |
| Murine RNase Inhibitor (Vazyme, catalog number: DD4102) | 40 U/μL | 2 |
| Pyrophosphatase, Inorganic (Vazyme, catalog number: DD4103) | 0.1 U/μL | 1 |
| Template DNA (SEQ ID NO: 286) | 0.5 μg/μL | 1 |

(continued)

| Component | Concentration | Volume (μL) |
|---|---|---|
| pH 6.8, HEPES buffer (containing 25 mM TCEP, 20 mM spermidine) | \ | 2 |
| T7 RNA polymerase | 300 U/μL | 1 |
| $MgCl_2$ | 200 mM | 2 |
| ddH2O | \ | Making up to 20 μL |

(2) Capillary electrophoresis was performed using a Qsep400 automated nucleic acid analyzer to detect the integrity of the saRNA product.

The detection results show that the variants in the K387A group and the DEL172+K387Y group can increase the integrity of the saRNA product by 3.1% and 4.9%, respectively, relative to the saRNA product obtained using the polymerase in the WT group.

## Example 4 Preparation of capped RNA by in-vitro transcription

(1) The enzyme stock solutions of the variants K387Y and DEL172+K387S were each diluted with a storage buffer (Vazyme, catalog number: DD4101) to an enzyme activity of 300 U/μL. The reaction system (20 μL) according to Table 7 was prepared into a MIX solution in an EP tube. The MIX solution was sub-packaged into an 8-tube strip, mixed uniformly, and centrifuged. The 8-tube strip was placed on a PCR instrument and the reaction was performed at 37°C for 1 h. Then 36 μL of magnetic beads were added, and the resulting mixture was mixed uniformly and then incubated at room temperature for 2 to 5 min. The mixture was placed on a magnetic rack to purify mRNA (Vazyme, catalog number: N412). After purification, the resulting product was transferred to an RNase-free centrifuge tube to obtain the purified mRNA.

Table 7: Reaction system

| Component | Concentration | Volume (μL) |
|---|---|---|
| ATP | 100 mM | 2 |
| GTP | 100 mM | 2 |
| CTP | 100 mM | 2 |
| m1ψ | 100 mM | 2 |
| Murine RNase Inhibitor (Vazyme, catalog number: DD4102) | 40 U/μL | 1 |
| Pyrophosphatase, Inorganic (Vazyme, catalog number: DD4103) | 0.1 U/μL | 1 |
| CleanCap AG (Trilink, catalog number: N-7113) | 5 mM | \ |
| Template DNA (SEQ ID NO: 285) | \ | 1 μg |
| 10×Transcription buffer (Vazyme, catalog number: DD4101R) | \ | 2 |
| T7 RNA polymerase | 300 U/μL | 1 |
| ddH2O | \ | Making up to 20 μL |

(2) Capping rate detection:

① The purified mRNA obtained in step (1) bound to a probe, with the reaction system as shown in Table 8 and the reaction conditions as shown in Table 9.

Table 8: Reaction system

| Component | 25 pmol volume |
|---|---|
| mRNA | 25 pmol |
| RNase-free $H_2O$ | (20-*V*) μL |

(continued)

| Component | 25 pmol volume |
|---|---|
| Probe (50 μM) (customized by GenScript) | 1 μL |
| Total system | 21 μL |

Table 9: Reaction conditions:

| Temperature | Time |
|---|---|
| 95°C | 2 min |
| 70 °C to 16°C | -0.1°C/s, ≈ 180 cycles |
| 16°C | ∞ |

② RNase H cleavage: the enzyme cleavage reaction system (Thermo Scientific, catalog number: EN0201) was prepared according to Table 10, mixed uniformly by complete vortex shaking, placed in a PCR instrument, and reacted at 25°C for 20 min.

Table 10: Reaction system

| Component | 25 pmol volume |
|---|---|
| Product from the previous step | 21 μL |
| RNase H Reaction Buffer (10×) | 3 μL |
| RNase H (5 U/μl) | 3 μL |
| RNase-free H$_2$O | 3 μL |
| Total system | 30 μL |

③ SA magnetic bead binding:

A. Cleaning of magnetic beads: 9 μL of SA magnetic beads were taken and added into a centrifuge tube, and the centrifuge tube was placed on a magnetic rack. After the solution became clear, the supernatant was aspirated and discarded using a pipettor. The centrifuge tube was removed from the magnetic rack. 200 μL of RNase-free H$_2$O was added for rinsing. The centrifuge tube was then placed on the magnetic rack. After the solution became clear, the supernatant was aspirated and discarded using a pipettor. Then 200 μL of RNase-free H$_2$O was added for rinsing again.
B. Reaction conditions: The centrifuge tube was removed from the magnetic rack, and the enzyme cleavage product was added to the SA magnetic beads. The resulting mixture was completely and uniformly mixed by pipetting 20-30 times using a pipettor, and then subjected to tumbling incubation at room temperature on a tumbling instrument for 30 min, so that the magnetic beads fully bound to the enzyme cleavage product.

④ Rinsing and elution:

A. The product from step ③ was placed on a magnetic rack for 2 to 3 min, and after the solution became clear, the supernatant was aspirated and discarded using a pipettor;
B. 200 μL of a rinsing liquid was added for rinsing (taking care to avoid pipetting the magnetic beads), the resulting mixture was left to stand for 0.5 to 1 min, and the supernatant was aspirated and discarded using a pipettor;
C. Step B was repeated;
D. The centrifuge tube was removed from the magnetic rack, 30 μL of an eluent was added, and the resulting mixture was uniformly mixed by pipetting 10-20 times using a pipettor, so that the magnetic beads were dispersed uniformly and fully eluted;
E. The eluted magnetic beads were placed in a PCR instrument, reacted at 85°C for 3 min, and then immediately placed on the magnetic rack, and after the solution became clear (0.5 to 1 min), the supernatant was pipetted and transferred to a new centrifuge tube, that is, the supernatant was the desired product;
F. The capping rate of the product from step E was detected by capillary electrophoresis, and the capping rate was calculated according to the following formula:

$$\text{Cap1 capping rate\%} = [\text{Cap1 peak area}/(\text{Uncap peak area} + \text{Cap1 peak area})] \times 100\%.$$

The detection results show that in the co-transcriptional capping reaction, the capping rate of the mRNA product in the WT (wild-type T7 RNA polymerase) group is only 87.2%, while the variant in the K387Y group can increase the capping rate of the mRNA product to 91.3%, and the variant in the DEL172+K387S group can increase the capping rate to 100%.

**Claims**

1. An RNA polymerase variant, having an amino acid sequence comprising, relative to SEQ ID NO: 1, one, two, three, four or five amino acid mutations at positions selected from D130, N171, K172, R173, Y178, R298, Y385, K387, D388 or F880, wherein the mutation type is a substitution or deletion, and the amino acid sequence of the variant has at least 97%, at least 98% or at least 99% sequence identity to SEQ ID NO: 1.

2. The variant according to claim 1, wherein the amino acid sequence of the variant has, relative to SEQ ID NO: 1, one amino acid mutation, and the position of the amino acid mutation is selected from N171, K172, R173, Y178, R298, Y385, K387, D388 or F880.

3. The variant according to claim 1, wherein the amino acid sequence of the variant comprises, relative to SEQ ID NO: 1,

   (1) a mutation at position K172, and also any one, two, three or four amino acid mutations at positions selected from D130, R173, Y178, R298, Y385, K387, D388 or F880, wherein the mutation type is a substitution or deletion; or
   (2) a mutation at site K173, and also any one, two or three amino acid mutations at positions selected from D130, Y178, R298, K387 or D388, wherein the mutation type is a substitution or deletion; or
   (3) any two or three amino acid mutations at positions selected from R298, Y385, K387 or D388, wherein the mutation type is a substitution.

4. The variant according to claim 3, wherein the variant comprises a mutation combination selected from any one of: K172+R173, D130+K172, K172+K387, K172+F880, K172+D388, K172+R298, D130+R173, R173+Y178, R173+D388, R173+R298, K387+R298, Y385+R298, D388+R298, Y385+K387, Y385+D388, K387+D388, K172+R173+Y385, K172+R173+D388, K172+R173+K387, K172+R173+F880, K172+R173+Y178, D130+K172+R173, D130+K172+Y178, D130+K172+K387, D130+R173+D388, K172+Y178+D388, D130+K172+D388, K172+K387+R298, Y385+K387+D388, K172+R173+Y385+F880, K172+R173+D388+F880, K172+R173+Y178+D388, D130+K172+R173+D388, D130+K172+R173+Y178, D130+R173+Y178+K387, D130+K172+Y178+D388, or D130+K172+R173+Y178+D388.

5. The variant according to claim 2, wherein

   1) the mutation type at position N171 is N171G;
   2) the mutation type at position K172 is selected from DEL172, K172A, K172G, K172E, K172D, K172H, K172Y, K172S, K172W, K172P, K172I, K172M, K172V, K172F, K172T, K172C, K172N or K172L;
   3) the mutation type at position R173 is selected from DEL173, R173A, R173C, R173G, R173E, R173D, R173H, R173Y, R173S, R173W, R173P, R173N or R173Q;
   4) the mutation type at position Y178 is Y178H;
   5) the mutation type at position R298 is R298A;
   6) the mutation type at position Y385 is selected from Y385A, Y385D or Y385E;
   7) the mutation type at position K387 is selected from K387Q, K387Y, K387S or K387A;
   8) the mutation type at position D388 is selected from D388A, D388G or D388L; and
   9) the mutation type at position F880 is selected from F880A, F880G or F880W.

6. The variant according to claim 3 or 4, wherein

   1) the mutation type at position D130 is D130E;

2) the mutation type at position K172 may be selected from DEL172, K172A or K172G;

3) the mutation type at position R173 may be selected from DEL173, R173A, R173G or R173C;

4) the mutation type at position Y178 may be selected from Y178H or Y178P;

5) the mutation type at position R298 is R298A;

6) the mutation type at position Y385 is Y385A;

7) the mutation type at position K387 may be selected from K387S, K387Y or K387G;

8) the mutation type at position D388 may be selected from D388Y, D388A or D388G; and

9) the mutation type at position F880 may be selected from F880A or F880Y.

7. The variant according to claim 1, wherein the amino acid sequence of the variant is set forth in any one of SEQ ID NOs: 2-141.

8. A nucleotide sequence encoding the variant according to any one of claims 1-7.

9. A method for preparing an RNA polymerase variant, comprising generating at least one RNA polymerase variant according to any one of claims 1-7 in a host cell, wherein the host cell contains an expression vector carrying the nucleotide sequence according to claim 8.

10. A method for reducing the generation of dsRNA impurities during the preparation of RNA by in-vitro transcription, comprising bringing a DNA template into contact with the variant according to any one of claims 1-7, and performing incubation in an in-vitro transcription system.

11. Use of at least one variant according to any one of claims 1-7 or the method according to claim 10 in the generation of RNA by in-vitro transcription.

12. A method for generating RNA by in-vitro transcription, comprising bringing a DNA template into contact with at least one variant according to any one of claims 1-7, and performing incubation in an in-vitro transcription system.

13. A method for generating capped RNA by in-vitro transcription, comprising bringing a DNA template into contact with at least one variant according to any one of claims 1-7 and a cap analog, and performing incubation in an in-vitro transcription reaction system.

14. A composition or kit, comprising at least one RNA polymerase variant according to any one of claims 1-7 and at least one buffer component.

FIG. 1